# EUROPEAN PATENT SPECIFICATION

(11) **EP 0 242 148 B1**
(45) Date of publication and mention of the grant of the patent: **13.01.1993**
(21) Application number: 87303183.5
(22) Date of filing: 10.04.1987
(51) Int. Cl.: C09B 61/00, C09B 67/54, A23L 1/275, C07C 403/00

(54) **Method for purification of carotene-containing concentrate**
Verfahren zur Reinigung eines Caroten enthaltenden Konzentrats
Méthode de purification d'un concentré contenant du carotène

(30) Priority: 15.04.1986 JP 86333/86; 06.10.1986 JP 237535/86
(43) Date of publication of application: 21.10.1987
(73) Proprietor: LION CORPORATION, Sumida-ku Tokyo (JP)
(72) Inventor: Hama, Itsuo, Chiba-shi Chiba (JP); Hara, Noboru, Setagaya-ku Tokyo (JP); Tanaka, Yoshiro, Ichikawa-shi Chiba (JP); Nakamura, Masayoshi, Chiba-shi Chiba (JP)
(74) Representative: Adams, William Gordon

(56) References cited:
- GB-A- 2 160 874
- JOURNAL OF THE ASSOCIATION OF OFFICIAL ANALYTICAL CHEMISTS, vol. 57, no. 3, May 1974, pages 511-512, Washington, US; F.W. QUACKENBUSH; #"Extraction and analysis of carotenoids in fresh plant materials"
- JRL. SCI. IND. RES., vol. 27, no. 8, August 1968, pages 306-315, IN; C. SIBBARAYAN et al.: "Carotenoids"

## Description

The present invention relates to a method for purification of a carotene concentrate by chromatography.

Carotene is widely used as a colorant for foods or substances having provitamin A action, and can be obtained by synthesis or recovery of carotene contained in natural fats and oils. As methods for recoverying carotene from a natural material, a method is known in which a natural fat and oil containing carotene is saponified, and carotene is then extracted as an unsaponified product with a solvent, and a method in which alcoholysis is effected by an addition of a lower alcohol to a natural fat and oil containing carotene to form a fatty acid lower alkyl ester, which is followed by vacuum distillation thereof to concentrate carotene in the vacuum residue.

Also, a method has been reported in which an oily substance containing carotene is brought into contact with a styrene-divinylbenzene copolymer resin to be adsorbed thereon, the resin is then brought into contact with an alcohol to separate the oily substance by elution, and thereafter, the resin is brought into contact with a carotene readily-soluble hydrophobic solvent to separate the carotene by elution (Japanese Unexamined Patent Publication (Kokai) No. 61-12657).

Further, the present applicant has proposed a method in which alcoholysis of a natural fat and oil containing carotene is effected with a lower monoalcohol, the oil phase containing carotene formed and additionally containing a fatty acid lower alkyl ester formed as the main component is collected, and at the same time, the oil phase is mixed with a hydrophilic solvent such as methanol, ethanol, isopropanol, acetone, and water to precipitate carotene, and the carotene thus-formed is collected (Japanese Patent Application No. 59-236013).

The present invention further purifies the carotene concentrate obtained by various methods, with the use of chromatography.

In the prior art, as the method for purification of carotene by using chromatography, investigations have been made only into high speed liquid chromatography for analysis in which calcium hydroxide is used as the filler and a hexane/acetone solvent mixture is used as the eluant, or the method in which active alumina is used as the filler and a hexane or hexane/benzene solvent mixture controlled in moisture is used as the eluant, but an industrially practiced example can not be found.

Generally speaking, in chromatography for analysis, the degree of purification and quantitative characteristic are the first object, and an expensive filler and complicated solvent mixtures have been used for enhancing the degree of separation. Such analytical methods involve the following problems when scaled-up as an industrial purification method.
(1) Due to the fine particle size of the filler employed, an extreme pressure loss occurs in a large scale column. On the other hand, when the particle size is made larger, the desired purification degree cannot be obtained.
(2) Due to the extremely small loaded amount of the sample, a large scall device having a large capacity must be used to obtain a desired treatment capacity.
(3) A long time is required for one batch.
(4) An enormous amount of the solvent must be used for the sample.

### SUMMARY OF THE INVENTION

Accordingly, the objects of the present invention are to eliminate the above-mentioned disadvantages of the prior art and to provide an industrially useful method for the purification of a carotene-containing concentrate by chromatography.

Other objects and advantages of the present invention will be apparent from the following description.

In accordance with the present invention, there is provided a method for purification of a carotene-containing concentrate by chromatography in which a carotene-containing concentrate is fed to a filler to adsorb the concentrate on the filler and the carotene component is subsequently eluted and collected by feeding an eluant to the filler, wherein silica gel or activated alumina pre-treated with a polar solvent selected from acetone, C₁-C₅ alcohols, C₁-C₃ alkyl esters of acetic acid, acetonitrile, dioxane, diethyl ether or mixtures thereof, and a non-polar solvent selected from C₅-C₂₀ hydrocarbons, carbon tetrachloride, carbon disulfide or mixtures thereof is used as said filler, and a solvent mixture of the above-mentioned polar solvent and the above-mentioned nonpolar solvent containing 0.005% to 5.0% by weight of said polar solvent is used as said eluant.

The present invention is now described in more detail by referring to the accompanying drawings.

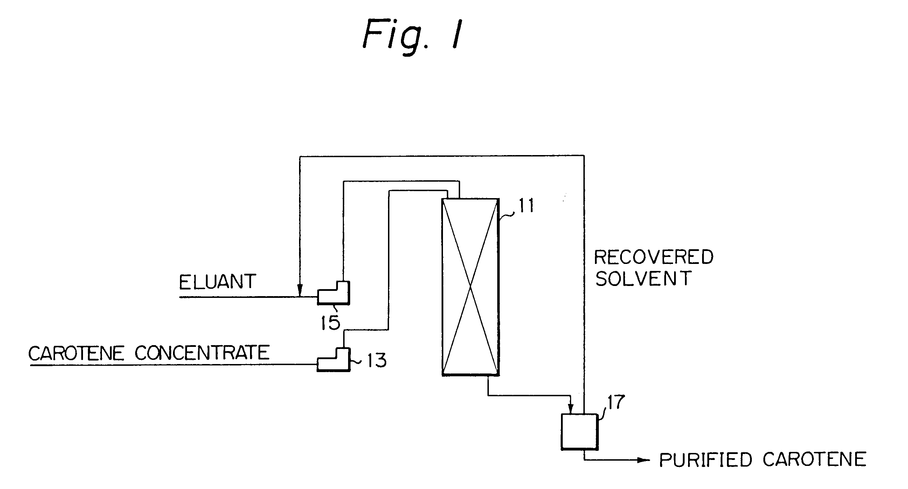

Figure 1 is a diagram of the present invention and shows a silica gel or activated alumina pretreated with a polar solvent and a nonpolar solvent is filled in the column 11. The particle size of the filler is not particularly limited, but particles of 50 to 600 µm in the case of silica gel, and particles of 300 to 1000 µm in the case of activated alumina, are preferably used.

According to the method of the present invention, a high purification degree can be obtained even when the particle size of the filler is made larger. The relatively larger particle sizes can be used produce an industrial advantage because of a smaller pressure loss. When calcium hydroxide or active alumina having particle sizes of about 1 to 10 µm, which were used as the filler in the analytical method of the prior art, are used as such in the device on an industrial scale, the problems as mentioned above will ensue. In contrast, silica gel or activated alumina having relatively larger particle sizes as mentioned above not only are inexpensive but also the carotene can be efficiently purified at a low pressure loss and at a high sample loading.

The filler used in the present invention is pre-treated with a polar solvent and a nonpolar solvent. The application of this pre-treatment allows the purification treatment to be carried out repeatedly under a stable elution state. This pretreatment can be practiced after filling the filler into the column 11, by feeding a polar solvent into the column so that the solvent is adsorbed to saturation point onto the filler, subsequently washing the filler with a nonpolar solvent or a mixture of a nonpolar solvent and a polar solvent, and completing the washing when the effluent has the same composition as the eluant.

Here, as the polar solvent, acetone, C₁-C₅ alcohols, c₁-C₃ alkyl acetate, acetonitrile, dioxane or diethyl ether or mixtures thereof are employed. On the other hand, as the nonpolar solvent, C₅-C₂₀ hydrocarbons (e.g., hexane), carbon tetrachloride or carbon disulfide or mixtures thereof are employed.

A liquid containing carotene concentrate is fed by a pump 13 to the column 11 filled with the pre-treated filler, so that the liquid is adsorbed onto the filler. As the carotene concentrate, an oily substance containing carotene at a high concentration is used, and the origin thereof is not particularly limited. However, preferably a product having an enhanced carotene concentration produced by carrying out alcoholysis of a natural fat and oil containing carotene such as palm oil with a lower monoalcohol and concentrating carotene in the resultant fatty acid lower alkyl ester by convenient means such as extraction, adsorption, distillation, is utilized.

The carotene-containing concentrate may be desirably diluted with the above-mentioned nonpolar solvent or a mixture thereof with the above polar solvent. The degree of dilution is not particularly limited if no undissolved matter exists, but preferably the saturated concentration of the carotene-containing concentrate is diluted in the solvent to be used, to shorten the sample feeding time in chromatography operation as much as possible.

Subsequently, a mixture of a polar solvent and a nonpolar solvent is fed by the feed pump 15 as the eluant to elute and collect the carotene component. The amount of the polar solvent added in the solvent mixture may be 0.005% to 5.0% by weight, preferably 0.01% to 0.5% by weight, particularly preferably 0.03% to 0.2% by weight. The kinds of the polar solvent and the nonpolar solvent to be used are the same as shown above in the pre-treatment of the filler.

The use of a nonpolar solvent as the eluant enables the carotene to be efficiently separated from other components such as fatty acid lower alkyl esters, and further, by the addition of a small amount of a polar solvent, the elution rate of the carotene portion can be rapidly enhanced while maintaining the purification degree of the carotene. As a result, the time needed for one batch can be shortened, and the amount of solvent used can be greatly reduced.

When the eluant is first fed, substances having a lower polarity than carotene are first eluted, and then the carotene portion is eluted. After collection of the carotene portion, by subsequent feeding of the same eluant, the components having a greater polarity than carotene are removed from the column to complete the operation of one batch.

By repeating the above-mentioned operation, the purification of carotene concentrate can be industrially effected. A pre-treatment of the filler with a polar solvent and a nonpolar solvent is conducted only when the filler is freshly filled, and the above-mentioned chromatography operation may be repeated thereafter.

Purified carotene is recovered from the collected liquid reservoir, which also functions as the solvent recovery tank 17, and the solvent is recovered and recycled.

When the above-mentioned purification operation by chromatography is repeated, substances having a high polarity which cannot be removed only by washing with the eluant gradually accumulate in the column and deteriorate the column performance. In carrying out purification by chromatography on an industrial scale, it is important to reduce production costs by utilizing such contaminated fillers repeatedly, by regeneration according to a simple operation.

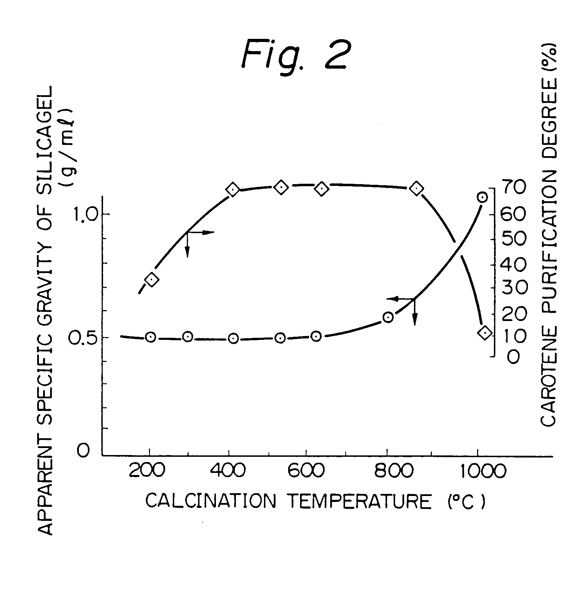

The present inventors have conducted intensive investigations into the regeneration of contaminated filler, and consequently, found that the fillers, both the silica gel and the activated alumina, can be regenerated to the state before use, and thus used repeatedly, by calcining the contaminated filler at a temperature of 400°C to 800°C. Figure 2 is a graph showing the relationship between the calcination temperature and the apparent specific gravity of the filler (silica gel) or the carotene purification degree. At a temperature of more than 800°C, ashing by dehydration of the silica gel is seen, while at a temperature of less than 400°C, a complete regeneration cannot be effected. The same behavior is also exhibited in the case of active alumina. The contaminated filler may be dried and then calcined as such or after washing with a polar solvent. Also, if necessary, the silica gel may be controlled to a desired water content by controlling the humidity after calcination.

The frequency of regeneration of the silica gel depends on the concentration of the polar substances in the carotene concentrate to be treated.

The method of the present invention is not limited to only the purification of carotene, but is also applicable as a chromatographic purification method of minute amounts of components, such as squalene, paraffin, tocopherol, contained in natural fat and oil.

Also, the system in which the filled column is previously pre-treated internally to the same atmosphere as the eluant and the column is washed with the same solvent as the eluant after chromatographic treatment, is also suitable as a general operation method of chromatography, because chromatographic purification and solvent recovery can be carried out with a very simple operation and under a stable state.

According to the present invention, by using silica gel or activated alumina treated with a polar solvent and a nonpolar solvent as the filler, and a nonpolar solvent added with a small amount of a polar solvent as the eluant, the carotene-containing concentrate obtained from natural fat and oil can be subjected to chromatographic purification repeatedly under an extremely stable eluted state, and the rate of elution of the carotene portion can be rapidly enhanced. Therefore, the time needed for one batch can be shortened, and the amount of the solvent used can be greatly reduced. Further, since the purification degree is not lowered even if the particle sizes of the filler are made larger, it is possible to efficiently perform the purification of carotene at a low pressure loss and at a high sample loading. Also, depending on the conditions, compared with the case when chromatography for analysis of the prior art is merely scaled-up, it is possible to realize a 1/100 pressure loss, an about 30 to 40-fold amount of sample loading, and a 1/10 amount of the solvent used.

Also, by diluting the carotene-containing concentrate with a nonpolar solvent or a nonpolar solvent - polar solvent mixture and using the same column washing solvent as the eluant, the recovery system of the solvent can be very much simplified. The solvent recovered by distillation, etc., can be utilized by recycling as such or with slight control of the composition for the eluant or the washing solvent.

### EXAMPLES

The present invention will now be further illustrated by, but is by no means limited to, the following Examples.

### Example 1

A device as shown in Fig. 1 was filled with 2.5 liters of spherical silica gel having a particle distribution of 50 to 600 µm according to the dry process, and subsequently, 150 g of acetone was fed into this column. Further, the column was washed with 15 liters of hexane, and it was confirmed that the acetone concentration in the effluent from the column bottom became 1000 ppm.

A crude palm oil containing 600 ppm of carotene (all calculated on trans-β-carotene, as hereinafter) was converted to a methyl ester with methanol, and further subjected to extracting concentration with methanol/water solvent mixture to obtain a carotene-containing concentrate containing 15% of carotene.

The carotene-containing concentrate (75 g) was diluted with 1425 g of hexane containing 1000 ppm of acetone and fed into the above column. Subsequently, elution was conducted with the use of hexane containing 1000 ppm of acetone, and the fractions from the point when the red portion began to flow out from the column bottom to completion were collected. The amount of the eluant fed in this operation was 2.5 cm/min based on a vacant column.

When the solvent was evaporated under reduced pressure from the collected red solution, 14 g of purified carotene with a carotene concentration of 72% was obtained. The carotene concentration degree was found to be 4.8-fold, and the carotene recovery 89.6%.

### Example 2

By use of the column used in Example 1, after elution of the red portion, the column was washed with 7 liters of hexane containing 1000 ppm of acetone, followed by the same chromatographic operation as in Example 1 using 75 g of the same carotene-containing concentrate as in Example 1 (carotene concentration 15%), whereby 15 g of a purified carotene with a carotene concentration of 69.2% was obtained. The carotene concentration degree was found to be 4.6-fold, and the carotene recovery 92.3%. The eluted state of the red portion peak exhibited substantially the same behavior as in Example 1.

### Example 3

When the operations of Examples 1 and 2 were repeated, the carotene concentration in the purified carotene was lowered to 56% (carotene concentration degree 3.7-fold) in the tenth operation.

Accordingly, the silica gel in the column was withdrawn, washed with 1 liter of acetone and then dried in air, followed by calcination at a temperature of 450°C for 6 hours. After the calcined silica gel was controlled to a water content of 0.7% with the addition of water, it was filled into the column to carry out chromatographic purification as in Example 1. As a result, 14.4 g of purified carotene with a carotene concentration of 70.2% (carotene concentration degree 4.7-fold, carotene recovery 90.0%) was obtained, thus exhibiting complete restoration of the function of the silica gel.

### Example 4

A device as shown in Fig. 1 was filled with 2.5 liters of spherical silica gel having a particle distribution of 50 to 600 µm according to the dry process, and subsequently, 50 g of methanol was fed into this column. Further, the column was washed with 10 liters of benzene, and it was confirmed that the methanol concentration in the effluent from the column bottom became 50 ppm.

The same carotene-containing concentrate as in Example 1 (75 g) was diluted with 675 g of benzene containing 50 ppm methanol and fed into the above column. Subsequently, elution was conducted with the use of benzene containing 50 ppm of methanol, and the fractions from the point when the red portion began to flow out from the column bottom to completion were collected. The amount of the eluant fed was 2.5 cm/min in terms of the line speed based on the vacant column.

When the solvent was evaporated under reduced pressure from the red solution collected, 14.5 g of a purified carotene with a carotene concentration of 69% was obtained. The carotene concentration degree was found to be 4.6-fold and the carotene recovery 88.9%.

### Example 5

By use of the same column as in Example 4, after elution of the red solution, the column was washed with 5 liters of benzene containing 50 ppm of methanol, followed by the same chromatographic operation as in Example 4 by using 75 g of the same carotene-containing concentrate (carotene concentration 15%) as in Example 4. As the result, 14 g of a purified carotene with a carotene concentration of 70.2% was obtained. The carotene concentration degree was found to be 4.7-fold and the carotene recovery 87.4%. The eluted state of the red portion peak exhibited substantially the same behavior as in Example 4.

### Example 6

To the same column as in Example 4, 2.5 liters of activated alumina having a particle size distribution of 300 to 1000 µm was filled according to the dry process, and then 25 g of diethyl ether was fed into this column. Further, the column was washed with 20 liters of n-heptane containing 50 ppm of diethyl ether, and it was confirmed that the diethyl ether concentration in the effluent from the column bottom became 50 ppm. A 75 g amount of the same carotene-containing concentrate (carotene concentration 15%) as in Example 4 was diluted with 1000 g of n-heptane containing 50 ppm of diethyl ether and fed into the above column. Subsequently, elution was conducted with the use of n-heptane containing 50 ppm of diethyl ether, and the fractions from the point when the red portion began to flow out from the column bottom to completion were collected.

When the solvent was evaporated under a reduced pressure from the collected red solution, 15.6 g of a purified carotene with a carotene concentration of 52% was obtained. The carotene concentration degree was found to be 3.5-fold, and the carotene recovery 72.1%.

## Claims

1. A method for purification of a carotene-containing concentrate by chromatography in which a liquid containing a carotene-containing concentrate is fed to a filler to adsorb the concentrate on the filler and the carotene component is subsequently eluted and collected by feeding an eluant to the filler, characterized in that silica gel or activated alumina pre-treated with a polar solvent selected from acetone, C₁-C₅ alcohols, C₁-C₃ alkyl esters of acetic acid, acetonitrile, dioxane, diethyl ether or mixtures thereof and a non-polar solvent selected from C₅-C₂₀ hydrocarbons, carbon tetrachloride, carbon disulfide or mixtures thereof is used as said filler, and a solvent mixture of the above-mentioned polar solvent and the above-mentioned nonpolar solvent containing 0.005% to 5.0% by weight of said polar solvent is used as said eluant.

2. A method as claimed in claim 1, wherein silica gel pre-treated with acetone and hexane is used as the filler and a mixture of acetone and hexane containing 0.005% to 1.0% by weight of acetone is used as the eluant.

## Patentansprüche

1. Verfahren zur Reinigung eines carotinhaltigen Konzentrats durch Chromatographie, wobei eine Flüssigkeit, die ein carotinhaltiges Konzentrat enthält, einem Füllmaterial zugeführt wird, um es auf diesem zu adsorbieren, und anschließend die Carotinkomponente durch Zuführen eines Eluiermittels zu dem Füllmaterial eluiert und gesammelt wird, dadurch gekennzeichnet, daß es sich bei diesem Füllmaterial um Kieselgel oder aktiviertes Aluminiumoxid, das mit einem aus Aceton, C₁-C₅ Alkoholen, C₁-C₃ Alkylestern der Essigsäure, Acetonitril, Dioxan, Diethylether sowie Mischungen aus diesen ausgewählten polaren Lösungsmitteln, und einem aus C₅-C₂₀ Kohlenwasserstoffen, Tetrachlorkohlenstoff, Schwefelkohlenstoff sowie Mischungen aus diesen ausgewählten nicht polaren Lösungsmitteln vorbehandelt ist, handelt und als Eluiermittel ein Lösungsmittelgemisch aus dem genannten polaren Lösungsmittel und dem genannten nicht polaren Lösungsmittel mit 0,005 bis 5,0 Gew.-% an dem polaren Lösungsmittel verwendet wird.

2. Verfahren nach Anspruch 1, worin mit Aceton und Hexan vorbehandeltes Kieselgel als Füllmaterial benutzt wird, und eine Mischung aus Aceton und Hexan mit 0,005 bis 1,0 Gew.-% Aceton, als Eluiermittel dient.

## Revendications

1. Méthode de purification d'un concentré contenant du carotène par chromatographie, dans laquelle une charge est alimentée par un liquide contenant un concentré contenant du carotène pour adsorber le concentré sur la charge, le carotène est par la suite dilué et recueilli en alimentant la charge par un diluant, caractérisé en ce qu'un gel de silice ou d'alumine activé pré-traité avec un solvant polaire choisi dans le groupe constitué par l'acétone, les alcools en C₁-C₅, les alkylesters en C₁-C₃ d'acide acétique, l'acétonitrile, le dioxane, le diéthyl éther ou des mélanges de ceux-ci et un solvant non polaire choisi dans le groupe constitué par les hydrocarbures en C₅-C₂₀, le tétrachlorure de carbone, le isulfure de carbone ou des mélanges de ceux-ci sont utilisés comme charge, et un mélange de solvants polaires mentionnés ci-dessus et de solvants non polaires mentionnés ci-dessus comprenant 0,005 % à 5,0 % en poids des dits solvants polaires est utilisé comme éluant.

2. Méthode selon la revendication 1, caractérisée en ce qu'un gel de silice pré-traité avec de l'acétone et de l'hexane est utilisé comme charge et un mélange d'acétone et d'hexane contenant 0,005 % à 1,0 % en poids d'acétone est utilisé comme éluant.
